(19) Europäisches Patentamt
European Patent Office
Office européen des brevets

(11) EP 4 129 341 A1

(12) **EUROPEAN PATENT APPLICATION**
published in accordance with Art. 153(4) EPC

(43) Date of publication:
**08.02.2023 Bulletin 2023/06**

(21) Application number: **21781950.7**

(22) Date of filing: **26.02.2021**

(51) International Patent Classification (IPC):
***A61K 47/02*** *(1990.01)* ***A61J 1/05*** *(1990.01)*
***A61K 9/08*** *(1974.07)* ***A61K 31/4704*** *(2000.01)*
***A61K 31/7084*** *(2000.01)* ***A61K 47/32*** *(1990.01)*
***A61P 27/02*** *(2000.01)*

(52) Cooperative Patent Classification (CPC):
**A61J 1/05; A61K 9/08; A61K 31/4704; A61K 31/7084; A61K 47/02; A61K 47/32; A61P 27/02**

(86) International application number:
**PCT/JP2021/007366**

(87) International publication number:
**WO 2021/199814 (07.10.2021 Gazette 2021/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.03.2020 JP 2020062252**

(71) Applicant: **Santen Pharmaceutical Co., Ltd.**
**Kita-ku**
**Osaka-shi**
**Osaka 530-8552 (JP)**

(72) Inventors:
- **MOMOKAWA, Yusuke**
**Ikoma-shi, Nara 630-0101 (JP)**
- **IIDA, Maki**
**Ikoma-shi, Nara 630-0101 (JP)**
- **ASADA, Hiroyuki**
**Inukami-gun, Shiga 522-0314 (JP)**
- **FUJISAWA, Toyomi**
**Ikoma-shi, Nara 630-0101 (JP)**

(74) Representative: **Müller-Boré & Partner**
**Patentanwälte PartG mbB**
**Friedenheimer Brücke 21**
**80639 München (DE)**

# (54) SILVER-SALT-CONTAINING OPHTHALMIC AQUEOUS COMPOSITION WITH WHICH RESIN CONTAINER IS FILLED

(57) The present invention relates to an ophthalmic aqueous composition containing a silver salt, and filled in a container made of a polyester-based resin, or a container made of a polyolefin-based resin excluding polypropylene. Besides, the present invention also relates to an ophthalmic preservative containing a silver salt, and filled in a container made of a polyester-based resin or a container made of a polyolefin-based resin excluding polypropylene, and a method for imparting, to an ophthalmic aqueous composition, preservative efficacy satisfying a criterion of Preservatives-Effectiveness Tests of The Japanese Pharmacopoeia, including adding a silver salt to the ophthalmic aqueous composition, and filling the ophthalmic aqueous composition in a container made of a polyester-based resin or a container made of a polyolefin-based resin excluding polypropylene. The present invention provides a preservative/system widely usable in ophthalmic aqueous compositions regardless of types of active ingredients and additives.



Processed by Luminess, 75001 PARIS (FR)

## Description

### TECHNICAL FIELD

The present invention relates to an ophthalmic aqueous composition containing a silver salt, and filled in a container made of a polyester-based resin, or a container made of a polyolefin-based resin excluding polypropylene.

### BACKGROUND ART

Since an ophthalmic aqueous composition is administered mainly by instillation, the ophthalmic aqueous composition is instilled into the eye several times a day in not a few cases, and from the viewpoint of convenience, is preferably filled in a container applicable to repeated instillation, namely, what is called a multi-dose eye drop container. When the composition is filled in a multi-dose eye drop container, benzalkonium chloride is generally added as a preservative for preventing bacterial contamination.

It is, however, known that use of benzalkonium chloride in a high concentration may possibly cause corneal disorder. Besides, it is known that benzalkonium chloride adsorbs onto a soft contact lens, and deforms a soft contact lens. In order to avoid such disadvantages, an ophthalmic aqueous composition containing no preservative is also used in treatment of an eye disease. For example, as described in a package insert for "Mucosta (registered trademark) ophthalmic suspension UD 2%" (NPL 1), no preservative is added to "Mucosta (registered trademark) ophthalmic suspension UD 2%". "Mucosta (registered trademark) ophthalmic suspension UD 2%" is, however, one-time disposable, and hence has a problem from the viewpoint of convenience described above.

On the other hand, there is a multi-dose ophthalmic aqueous composition containing a preservative safer than a benzalkonium chloride. For example, as described in a package insert for "Diquas (registered trademark) ophthalmic solution 3%" (NPL 2), not benzalkonium chloride generally used as a preservative but chlorhexidine gluconate is added to "Diquas (registered trademark) ophthalmic solution 3%". It is noted that Japanese Patent Laying-Open No. 2017-2036 (PTL 1) describes that chlorhexidine gluconate does not deform a soft contact lens. It is, however, not clear whether or not chlorhexidine gluconate can be always used instead of benzalkonium chloride regardless of types of active ingredients and additives contained in an ophthalmic aqueous composition.

A package insert for a silver nitrate ophthalmic solution "Born Happy (registered trademark)" (NPL 3) describes that the silver nitrate ophthalmic solution is used for the treatment of conjunctivitis gonorrhoica neonatorum. NPL 3 does not, however, describe that silver nitrate can be used as a preservative for an ophthalmic aqueous composition. Besides, Japanese National Patent Publication No. 2016-507469 (PTL 2) discloses an emulsion composition containing difluprednate and an antibacterial metal, and describes that an example of the antibacterial metal includes a silver salt, and that the emulsion composition can be formed as an ophthalmic composition. PTL 2 neither discloses nor suggests, however, a container of which material should be used for filling the composition.

### CITATION LIST

### PATENT LITERATURE

PTL 1: Japanese Patent Laying-Open No. 2017-2036
PTL 2: Japanese National Patent Publication No. 2016-507469

### NON PATENT LITERATURE

NPL 1: Package insert for "Mucosta (registered trademark) ophthalmic suspension UD 2%"
NPL 2: Package insert for "Diquas (registered trademark) ophthalmic solution 3%"
NPL 3: Package insert for a silver nitrate ophthalmic solution "Born Happy (registered trademark)"

### SUMMARY OF INVENTION

### TECHNICAL PROBLEM

An object of the present invention is to provide a preservative/system widely usable in ophthalmic aqueous

compositions regardless of types of active ingredients and additives.

SOLUTION TO PROBLEM

The present inventors have made earnest studies to solve the above-described problem, and as a result, have found that an ophthalmic aqueous composition containing a silver salt and filled in a container made of a polyester-based resin or a container made of a polyolefin-based resin excluding polypropylene has sufficient preservative efficacy over a long period of time, and thus, the present invention was accomplished.

Besides, the present inventors have found that a silver salt does not deform a soft contact lens (SCL), and hence the ophthalmic aqueous composition of the present invention can be administered by instillation to an SCL wearing eye.

Specifically, the present invention relates to the following:

(1) An ophthalmic aqueous composition containing a silver salt, the ophthalmic aqueous composition being filled in a container made of a polyester-based resin, or a container made of a polyolefin-based resin excluding polypropylene (hereinafter also referred to as the "present ophthalmic aqueous composition").
(2) The ophthalmic aqueous composition according to (1), in which the polyester-based resin is polyethylene terephthalate.
(3) The ophthalmic aqueous composition according to (1), in which the polyolefin-based resin is polyethylene.
(4) The ophthalmic aqueous composition according to any one of (1) to (3), in which a concentration of the silver salt in the ophthalmic aqueous composition is 0.001% (w/v) or less.
(5) The ophthalmic aqueous composition according to any one of (1) to (4), in which a concentration of the silver salt in the ophthalmic aqueous composition is 0.000003 to 0.0003% (w/v).
(6) The ophthalmic aqueous composition according to any one of (1) to (4), in which a concentration of the silver salt in the ophthalmic aqueous composition is 0.00001 to 0.0001 % (w/v).
(7) The ophthalmic aqueous composition according to any one of (1) to (4), in which a concentration of the silver salt in the ophthalmic aqueous composition is 0.00002 to 0.0001 % (w/v).
(8) The ophthalmic aqueous composition according to any one of (1) to (7), further containing an ionic tonicity agent.
(9) The ophthalmic aqueous composition according to any one of (1) to (8), in which the container made of a polyester-based resin or the container made of a polyolefin-based resin is a multi-dose eye drop container.
(10) The ophthalmic aqueous composition according to any one of (1) to (9), in which the ophthalmic aqueous composition is administered by instillation.
(11) The ophthalmic aqueous composition according to any one of (1) to (10), in which the ophthalmic aqueous composition is administered by instillation to a soft contact lens wearing eye.
(12) The ophthalmic aqueous composition according to any one of (1) to (11), in which the silver salt is silver nitrate.
(13) The ophthalmic aqueous composition according to any one of (1) to (12), containing an active ingredient.
(14) The ophthalmic aqueous composition according to (13), in which the active ingredient is rebamipide, diquafosol, or a salt thereof.
(15) The ophthalmic aqueous composition according to (13), in which the active ingredient is sirolimus or a salt thereof.
(16) The ophthalmic aqueous composition according to (13), containing an active ingredient excluding sirolimus or a salt thereof.
(17) An ophthalmic aqueous composition containig rebamipide, polyvinylpyrrolidone, and silver nitrate, the ophthalmic aqueous composition being filled in a multi-dose polyethylene eye drop container.
(18) An ophthalmic aqueous composition containing diquafosol sodium, polyvinylpyrrolidone, and silver nitrate, the ophthalmic aqueous composition being filled in a multi-dose polyethylene eye drop container.
(19) An ophthalmic aqueous composition containing sirolimus, a surfactant, and silver nitrate, the ophthalmic aqueous composition being filled in a multi-dose polyethylene eye drop container.
(20) An ophthalmic aqueous composition containing 0.00001 to 0.0001% (w/v) of silver nitrate, the ophthalmic aqueous composition being filled in a multi-dose polyethylene terephthalate eye drop container.
(21) An ophthalmic aqueous composition containing 0.00002 to 0.0001% (w/v) of silver nitrate, the ophthalmic aqueous composition being filled in a multi-dose polyethylene terephthalate eye drop container.

The present invention further relates to the following:

(22) An ophthalmic preservative comprising a silver salt, the ophthalmic preservative being filled in a container made of a polyester-based resin or a container made of a polyolefin-based resin excluding polypropylene (hereinafter also referred to as the "present ophthalmic preservative").
(23) A method for imparting, to an ophthalmic aqueous composition, preservative efficacy satisfying a criterion of

Preservatives-Effectiveness Tests of The Japanese Pharmacopoeia, including adding a silver salt to the ophthalmic aqueous composition, and filling the ophthalmic aqueous composition in a container made of a polyester-based resin or a container made of a polyolefin-based resin excluding polypropylene (hereinafter also referred to as the "present method").

## ADVANTAGEOUS EFFECTS OF INVENTION

The present ophthalmic aqueous composition has sufficient preservative efficacy over a long period of time, and hence can be formed into a multi-dose eye drop, and can be administered by instillation to an SCL wearing eye.

## DESCRIPTION OF EMBODIMENTS

In the present invention, examples of a silver salt include silver nitrate, silver sulfate, silver chloride, silver bromide, silver oxide, silver acetate, silver carbonate, silver citrate, silver lactate, silver phosphate, silver oxalate, silver thiosulfate, and silver protein, and the silver salt preferably means silver nitrate.

A concentration of the silver salt contained in the present ophthalmic aqueous composition is preferably 1% (w/v) or less, more preferably 0.1% (w/v) or less, further preferably 0.01% (w/v) or less, particularly preferably 0.001% (w/v) or less, and most preferably 0.0001% (w/v) or less. Besides, the concentration of the silver salt contained in the present ophthalmic aqueous composition is preferably 0.0000001% (w/v) or more, more preferably 0.000001% (w/v) or more, further preferably 0.000003% (w/v) or more, and most preferably 0.00001% (w/v) or more. From the viewpoint of attaining sufficient preservative efficacy without being affected by an active ingredient, an additive and the like contained in the present ophthalmic aqueous composition, the concentration of the silver salt contained in the present ophthalmic aqueous composition is also preferably 0.00002% (w/v) or more. A concentration range of the silver salt contained in the present ophthalmic aqueous composition is preferably 0.0000001 to 0.01% (w/v), more preferably 0.000001 to 0.001% (w/v), further preferably 0.000003 to 0.0003% (w/v), and most preferably 0.00001 to 0.0001% (w/v). Besides, from the viewpoint of attaining sufficient preservative efficacy without being affected by an active ingredient, an additive and the like contained in the present ophthalmic aqueous composition, the concentration range of the silver salt contained in the present ophthalmic aqueous composition is preferably 0.00002 to 0.01% (w/v), more preferably 0.00002 to 0.001% (w/v), further preferably 0.00002 to 0.0003% (w/v), and most preferably 0.00002 to 0.0001% (w/v).

In the present invention, the ophthalmic aqueous composition means an aqueous composition topically administered to the eye of a subject, and means, for example, an aqueous composition administered by instillation or topical injection into the eye. The ophthalmic aqueous composition is preferably an aqueous composition to be administered by instillation to the eye of a subject, and is designated also as an eye drop.

In the present invention, an aqueous composition means a composition using water as a base, and properties thereof do not matter. The aqueous composition encompasses a solution (aqueous solution), a suspension (aqueous suspension) and an emulsion using water as a base.

In the present invention, the term "container made of a polyester-based resin" means a container in which at least a portion to be in contact with the aqueous composition is made of a polyester-based resin. Accordingly, for example, a container in which a polyester-based resin layer is provided in an inner layer to be in contact with the ophthalmic aqueous composition with a layer of another material resin or the like laminated outside corresponds to the "container made of a polyester-based resin". Here, dicarboxylic acid and diol contained in the polyester-based resin are not particularly limited, and example of the dicarboxylic acid include phthalic acid, terephthalic acid, and 2,6-naphthalenedicarboxylic acid, and examples of the diol include ethylene glycol, 1,3-propanediol, 1,4-butanediol, 1,4-cyclohexanedimethanol, and bisphenol. Besides, the polyester-based resin may be a polymer having a single type of polyester unit, or a polymer having a plurality of types of polyester units. In the polymer having a plurality of types of polyester units, the polymerization method is not especially limited, and may be random polymerization or block polymerization. Besides, the tacticity is not especially limited.

Examples of the polyester-based resin include homopolyesters such as polyalkylene terephthalate (such as polyethylene terephthalate, and polybutylene terephthalate), polyalkylene naphthalate (such as polyethylene naphthalate, and polybutylene naphthalate), polycycloalkylene terephthalate (such as poly(1,4-cyclohexylenedimethylene terephthalate)), and polyarylate (such as a resin containing bisphenol and phthalic acid), copolyesters containing these homopolyester units as a principal component, and copolymers of the homopolyesters, and one of or a combination of two or more of these can be used.

The polyester-based resin most preferable in the present invention is polyethylene terephthalate.

In the present invention, being made of the polyester-based resin means that the polyester-based resin is contained in at least a part of the material, and for example, a mixture (polymer alloy) of two or more resins, that is, the polyester-based resin and another resin, is encompassed in the polyester-based resin.

In the present invention, the term "container made of a polyolefin-based resin" means a container in which at

least a portion to be in contact with the aqueous composition is made of a polyolefin-based resin. Accordingly, for example, a container in which a polyolefin-based resin layer is provided in an inner layer to be in contact with the ophthalmic aqueous composition with a layer of another material resin or the like laminated outside corresponds to the "container made of a polyolefin-based resin". Here, the polyolefin-based resin is not especially limited, and may be a polymer of a single type of monomer (homopolymer) or a copolymer of a plurality of types of monomers. In the copolymer, the polymerization method is not especially limited, and may be random polymerization or block polymerization. Besides, the tacticity is not especially limited.

Examples of the polyolefin-based resin include polyethylene, cyclic polyolefin, poly(4-methylpentene), polytetrafluoroethylene, an ethylene/propylene copolymer, an ethylene/$\alpha$-olefin copolymer, an ethylene/acrylic acid copolymer, an ethylene/methacrylic acid copolymer, an ethylene/vinyl acetate copolymer, and an ethylene/ethyl acrylate copolymer, and one of or a combination of two or more of these can be used. Besides, specific examples of the polyethylene include low density polyethylene (including linear low density polyethylene), high density polyethylene, and middle density polyethylene.

The polyolefin-based resin most preferable in the present invention is polyethylene, and low density polyethylene or high density polyethylene is preferred. In general, polypropylene is one of polyolefin-based resins, but when the present ophthalmic aqueous composition is filled in a polypropylene container, the silver salt adsorbs onto the container and sufficient preservative efficacy cannot be assured, and therefore, polypropylene is excluded from the materials of the resin container in which the present ophthalmic aqueous composition is filled.

In the present invention, the term "being made of the polyolefin-based resin" means that the polyolefin-based resin is contained in at least a part of the material, and for example, a mixture (polymer alloy) of two or more resins, that is, the polyolefin-based resin and another resin, is encompassed in "being made of the polyolefin-based resin".

In the present invention, the resin container is preferably an eye drop container, and in particular, a what is called multi-dose eye drop container capable of repeated instillation of an aqueous composition filled therein by opening/closing the container is most preferred.

Soft contact lenses (SCL) are classified into 4 groups in accordance with Notification No. 645 issued on March 31, 1999 by Pharmaceutical and Food Safety Bureau, "Guidance of documents to be attached to application for approval of manufacture (import) of soft contact lenses and soft contact lens disinfectant solutions". Specifically, the SCLs are classified into Group I (nonionic ones having a water content less than 50%), Group II (nonionic ones having a water content of 50% or more), Group III (ionic ones having a water content less than 50%), and Group IV (ionic ones having a water content of 50% or more), and one in which mol% of a monomer having an anion among constituent monomers of a raw material polymer is 1% or more is ionic, and one in which the mol% is less than 1% is nonionic. Besides, examples of the soft contact lens include soft contact lenses containing, as a principal component, 2-hydroxyethylmethacrylate (HEMA), (polyethylene glycol) monomethacrylate (PEGMA), glycerol methacrylate (GMA), N,N-dimethylacrylamide (DMA), vinyl alcohol (VA), N-vinylpyrrolidone (NVP or VP), methacrylic acid (MAA), a fluorine-containing methacrylate-based compound, a silicon-containing methacrylate-based compound, silicone hydrogel, and cycloalkyl methacrylate.

In the present invention, the term "being administered by instillation to a soft contact lens wearing eye" means that the present ophthalmic aqueous composition can be administered by instillation with a soft contact lens worn.

As described below, the present ophthalmic aqueous composition may adsorb also onto a polyolefin-based resin when the concentration of the silver salt to be added is lowered, and therefore, an ionic tonicity agent can be added for suppressing the adsorption.

An amount of the ionic tonicity agent to be added to the present ophthalmic aqueous composition is not especially limited as long as the present ophthalmic aqueous composition is isotonized with the amount, and for example, 0.1 to 0.9% (w/v) of the ionic tonicity agent can be added to the present ophthalmic aqueous composition.

In the present invention, examples of the "ionic tonicity agent" include sodium chloride, potassium chloride, calcium chloride, and magnesium chloride.

Besides, to the present ophthalmic aqueous composition, polyvinylpyrrolidone can be added. It is noted that polyvinylpyrrolidone means a polymer compound obtained by polymerization of N-vinyl-2-pyrrolidone, and is designated also as povidone.

The polyvinylpyrrolidone to be contained in the present ophthalmic aqueous composition has a K value of preferably 17 or more, more preferably 17 to 120, further preferably 25 to 120, and particularly preferably 30 to 120.

In the present invention, examples of the "polyvinylpyrrolidone" include polyvinylpyrrolidone K15 (PVP K15), polyvinylpyrrolidone K17 (PVP K17), polyvinylpyrrolidone K25 (PVP K25), polyvinylpyrrolidone K30 (PVP K30), polyvinylpyrrolidone K40 (PVP K40), polyvinylpyrrolidone K50 (PVP K50), polyvinylpyrrolidone K60 (PVP K60), polyvinylpyrrolidone K70 (PVP K70), polyvinylpyrrolidone K80 (PVP K80), polyvinylpyrrolidone K85 (PVP K85), polyvinylpyrrolidone K90 (PVP K90), and polyvinylpyrrolidone K120 (PVP K120).

It is noted that the K value of polyvinylpyrrolidone is a viscosity property value correlated to a molecular weight, and is a numerical value calculated by applying a relative viscosity value (25°C) measured with a capillary viscometer

to the following expression (1) of Fikentscher:
[Expression 1]

$$K=\frac{1.5\log\eta_{rel}-1}{0.15+0.003c}+\frac{[300c\log\eta_{rel}+2(c+1.5\log\eta_{rel})]^{1/2}}{0.15c+0.003c^{2}} \quad (1)$$

In expression (1), ηrel is a relative viscosity of a polyvinylpyrrolidone aqueous solution to water, and c is a polyvinylpyrrolidone concentration (%) in the polyvinylpyrrolidone aqueous solution.

Here, the K value is 90 to 108% of a display K value in accordance with description of the K value of "Povidone" in the 17th edition of the Japanese Pharmacopoeia, and hence, for example, "K30" means that the viscosity property value (K value) calculated by applying expression (1) described above is in a range of 27 to 32.4, and "K90" means that the viscosity property value (K value) calculated by applying expression (1) described above is in a range of 81 to 97.2.

The polyvinylpyrrolidone to be contained in the present ophthalmic aqueous composition may be a single one of polyvinylpyrrolidones, or an arbitrary combination of two or more polyvinylpyrrolidones having different K values.

Besides, in order to retain dispersibility and redispersibility of an active ingredient contained in the present ophthalmic aqueous composition for suppressing agglomeration, a surfactant can be compounded in the present ophthalmic aqueous composition.

In the present ophthalmic aqueous composition, a surfactant usable as an additive of a pharmaceutical can be appropriately compounded, and examples include a cationic surfactant, an anionic surfactant, an amphoteric surfactant, and a nonionic surfactant, and the surfactant may be a hydrate or a solvate of any of these.

In the present invention, examples of the "cationic surfactant" include amine salts such as an alkylamine salt, an alkylamine polyoxyethylene adduct, a fatty acid triethanolamine monoester salt, an acylaminoethyl diethylamine salt, a fatty acid polyamine condensate, alkylimidazoline, 1-acylaminoethyl-2-alkylimidazoline, and 1-hydroxyethyl-2-alkylimidazoline; and ammonium salts such as benzalkonium chloride, benzethonium chloride, and chlorhexidine gluconate.

In the present invention, examples of the "anionic surfactant" include sulfonates such as alkylbenzene sulfonate, α-olefin sulfonate, and α-sulfo fatty acid ester salt; sulfate salts such as an alkyl sulfate salt, and polyoxyethylene alkyl sulfate salt; and phosphates such as sodium polyoxyethylene cetyl ether phosphate.

In the present invention, examples of the "nonionic surfactant" include polyoxyethylene fatty acid esters such as polyoxyl 40 stearate; polyoxyethylene sorbitan fatty acid esters such as polysorbate 80, polysorbate 60, polysorbate 40, polyoxyethylene sorbitan monolaurate, polyoxyethylene sorbitan triolate, and polysorbate 65; polyoxyethylene hardened castor oils such as polyoxyethylene hardened castor oil 10, polyoxyethylene hardened castor oil 40, polyoxyethylene hardened castor oil 50, and polyoxyethylene hardened castor oil 60; polyoxyl castor oils such as polyoxyl 5 castor oil, polyoxyl 9 castor oil, polyoxyl 15 castor oil, polyoxyl 35 castor oil, and polyoxyl 40 castor oil; polyoxyethylene polyoxypropylene glycols such as polyoxyethylene (160) polyoxypropylene (30) glycol, polyoxyethylene (42) polyoxypropylene (67) glycol, polyoxyethylene (54) polyoxypropylene (39) glycol, polyoxyethylene (196) polyoxypropylene (67) glycol, and polyoxyethylene (20) polyoxypropylene (20) glycol; sucrose fatty acid esters such as sucrose stearate; and tocopherol polyethylene glycol 1000 succinate (vitamin E TPGS).

To the present ophthalmic aqueous composition, an additive can be added in addition to the ionic tonicity agent, the polyvinylpyrrolidone, and the surfactant. For example, an additive can be prepared by appropriately selecting, if necessary, from nonionic tonicity agents such as glycerin, propylene glycol, polyethylene glycol, sorbitol, mannitol, trehalose, maltose, and sucrose; buffers such as sodium phosphate, sodium hydrogen phosphate, a sodium hydrogen phosphate hydrate, sodium dihydrogen phosphate, a sodium citrate hydrate, sodium acetate, and epsilon-aminocaproic acid; stabilizers such as disodium edetate and a disodium edetate hydrate; antioxidants such as ascorbic acid; thickening agents (also designated as thickeners) such as a carboxyl vinyl polymer, hydroxyethylcellulose, and hydroxypropyl methylcellulose (hypromellose); and pH adjustors such as hydrochloric acid and sodium hydroxide, and the additive may have pH falling in an acceptable range for ophthalmic preparations, and usually preferably in a range of 4 to 8.

The present ophthalmic aqueous composition can contain an active ingredient. Examples of the active ingredient to be contained in the present ophthalmic aqueous composition (hereinafter, simply referred to also as the "present active ingredient") include a dry eye/corneal disease therapeutic agent, an antiallergic agent, a steroidal anti-inflammatory agent, a nonsteroidal anti-inflammatory agent, an intraocular pressure lowering agent, an antiviral agent, and an antibacterial agent.

Specific examples of the dry eye/corneal disease therapeutic agent include diquafosol, rebamipide, and salts thereof.

Other specific examples of the dry eye/corneal disease therapeutic agent include cyclosporine, lifitegrast, and salts thereof.

Specific examples of the antiallergic agent include olopatadine, levocabastine, ketotifen, and salts thereof.

Specific examples of the steroidal anti-inflammatory agent include fluorometholone, hydrocortisone, triamcinolone, fluorocinolone, dexamethasone, betamethasone, and salts thereof.

Specific examples of the nonsteroidal anti-inflammatory agent include indomethacin, bromfenac, diclofenac, olopatadine, levocabastine, ketotifen, and salts thereof.

Specific examples of the intraocular pressure lowering agent include brimonidine, dorzolamide, brinzolamide, timolol, carteolol, bimatoprost, latanoprost, travoprost, ripasudil, and salts thereof.

Specific examples of the antiviral agent include acyclovir and salts thereof.

Specific examples of the antibacterial agent include gatifloxacin, moxifloxacin, tosufloxacin, and salts thereof.

In addition to those described above, other examples of the present active ingredient include sirolimus, and salts thereof.

The present active ingredient is preferably diquafosol, rebamipide, sirolimus, or a salt thereof, and diquafosol sodium, rebamipide (free form), and sirolimus (free form) are particularly preferred.

The diquafosol used in the present invention is a compound represented by the following formula:

[Formula 1]

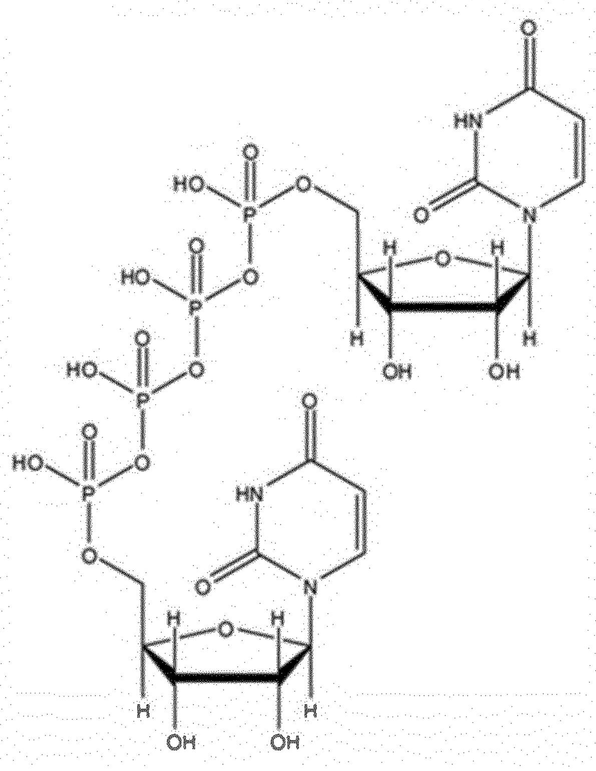

The rebamipide used in the present invention is a compound represented by the following formula:

[Formula 2]

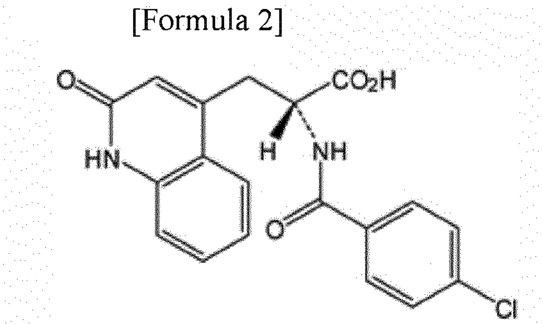

and the enantiomer

The sirolimus used in the present invention is a compound represented by the following formula:

[Formula 3]

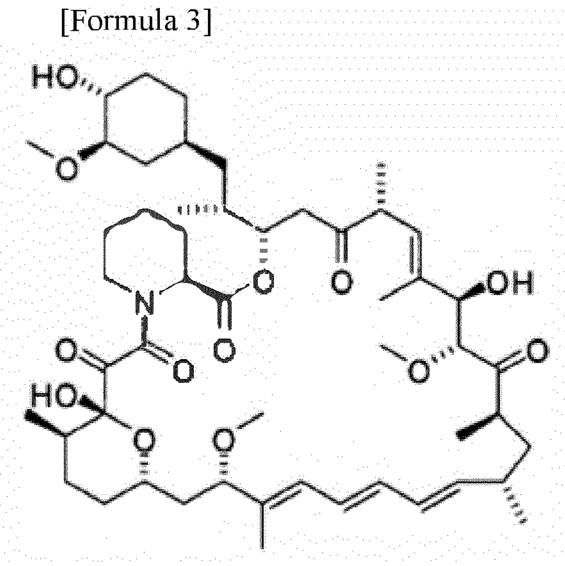

The salts of the present active ingredients are not especially limited as long as they are pharmaceutically acceptable salts, and examples include a salt with an inorganic acid such as hydrochloric acid, hydrobromic acid, hydroiodic acid, nitric acid, sulfuric acid, or phosphoric acid; a salt with an organic acid such as acetic acid, fumaric acid, maleic acid, succinic acid, citric acid, tartaric acid, adipic acid, gluconic acid, glucoheptonic acid, glucuronic acid, terephthalic acid, methanesulfonic acid, lactic acid, hippuric acid, 1,2-ethanedisulfonic acid, isethionic acid, lactobionic acid, oleic acid, pamoic acid, polygalacturonic acid, stearic acid, tannic acid, trifluoromethanesulfonic acid, benzenesulfonic acid, p-toluenesulfonic acid, lauryl sulfate ester, methyl sulfate, naphthalenesulfonic acid, or sulfosalicylic acid; a quaternary ammonium salt with methyl bromide, or methyl iodide; a salt with a halogen ion such as a bromine ion, a chlorine ion, or an iodine ion; a salt with an alkali metal such as lithium, sodium, or potassium; a salt with an alkaline earth metal such as calcium, or magnesium; a metal salt with iron, zinc or the like; a salt with ammonia; and a salt with an organic amine such as triethylenediamine, 2-aminoethanol, 2,2-iminobis(ethanol), 1-deoxy-1-(methylamino)-2-D-sorbitol, 2-amio-2-(hydroxymethyl)-1,3-propanediol, procaine, or N,N-bis(phenylmethyl)-1,2-ethanediamine.

In the present invention, the solvate such as a hydrate of the present active ingredient is encompassed in the salt of the present active ingredient.

In the present invention, when the present active ingredient or a salt thereof has a geometric isomer or an optical isomer, the isomer and a salt thereof are encompassed in the scope of the present invention. Besides, when the present active ingredient or a salt thereof has a proton tautomer, the tautomer and a salt thereof are encompassed in the scope of the present invention.

In the present invention, when the present active ingredient or a salt thereof has crystal polymorphism or a crystal polymorphism group (crystal polymorphism system), these crystal polymorphism and crystal polymorphism group (crystal polymorphism system) are encompassed in the scope of the present invention. Here, the crystal polymorphism group (crystal polymorphism system) means crystal forms at the respective stages in various changes of the crystal form caused depending on the conditions and states of preparation, crystallization, preservation, and the like of these crystals (note that a state in the form of a preparation is also included in these states), and the whole processes.

In the present invention, the "diquafosol or a salt thereof" is preferably tetrasodium salt of diquafosol represented by the following formula (hereinafter, simply referred to also as "diquafosol sodium"):

[Formula 4]

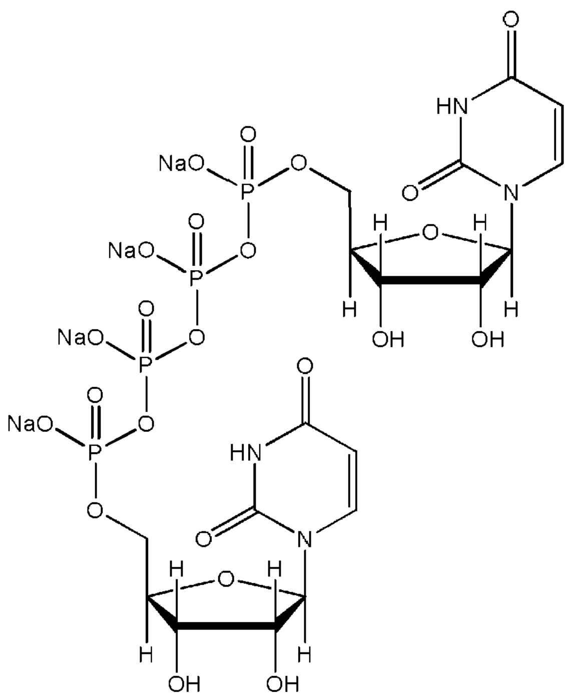

In the present invention, the "rebamipide or a salt thereof" is preferably rebamipide (free form).

In the present invention, the "sirolimus or a salt thereof" is preferably sirolimus (free form).

When the present active ingredient is diquafosol sodium, polyvinylpyrrolidone can be added to the present ophthalmic aqueous composition for purposes of reducing instillation frequency. In this case, the polyvinylpyrrolidone has a K value of preferably 60 to 120, more preferably 60 to 90, and particularly preferably 90. Accordingly, when the present active ingredient is diquafosol sodium, it is preferable to add, to the present ophthalmic aqueous composition, polyvinylpyrrolidone K60, polyvinylpyrrolidone K70, polyvinylpyrrolidone K80, polyvinylpyrrolidone K85, polyvinylpyrrolidone K90, or polyvinylpyrrolidone K120, and it is particularly preferable to add polyvinylpyrrolidone K90.

When the present active ingredient is diquafosol sodium, the concentration of the polyvinylpyrrolidone to be added to the present ophthalmic aqueous composition is preferably 0.1 to 10% (w/v), more preferably 0.1 to 5% (w/v), and further preferably 1 to 5% (w/v).

Specifically, when the present active ingredient is diquafosol sodium, the present ophthalmic aqueous composition can be formed as an ophthalmic aqueous composition containing diquafosol sodium, polyvinylpyrrolidone, and silver nitrate, and filled in a multi-dose eye drop container made of polyethylene.

As described above, various additives can be added to the present ophthalmic aqueous composition, and when the present active ingredient is diquafosol sodium, it is preferable to add, to the present ophthalmic aqueous composition, an ionic tonicity agent such as sodium chloride, a buffer such as a sodium hydrogen phosphate hydrate, a stabilizer such as a disodium edetate hydrate, a pH adjustor and the like in addition to the polyvinylpyrrolidone.

When the present active ingredient is rebamipide, an average particle size (D50) of rebamipide to be contained in the present ophthalmic aqueous composition can be set to preferably 0.01 to 10 μm, more preferably 0.05 to 5 μm, further preferably 0.1 to 3 μm, and particularly preferably 0.5 to 1 μm by adding polyvinylpyrrolidone to the present ophthalmic aqueous composition. In this case, the polyvinylpyrrolidone has a K value of preferably 17 to 90, more preferably 17 to 60, and particularly preferably 30. Accordingly, when the present active ingredient is rebamipide, it is preferable to add, to the present ophthalmic aqueous composition, polyvinylpyrrolidone K30, polyvinylpyrrolidone K40, polyvinylpyrrolidone K50, or polyvinylpyrrolidone K60, and it is particularly preferable to add polyvinylpyrrolidone K30.

When the present active ingredient is rebamipide, a concentration of the polyvinylpyrrolidone to be added to the present ophthalmic aqueous composition is preferably 0.1 to 2% (w/v), more preferably 0.5 to 2% (w/v), further preferably 1 to 2% (w/v), and most preferably 2% (w/v).

Specifically, when the present active ingredient is rebamipide, the present ophthalmic aqueous composition can be formed as an ophthalmic aqueous composition containing rebamipide, polyvinylpyrrolidone, and silver nitrate, and filled in a multi-dose eye drop container made of polyethylene.

When the present active ingredient is rebamipide, a carboxyl vinyl polymer can be added for purposes of improving the viscosity of the present ophthalmic aqueous composition. In this case, a concentration of the carboxyl vinyl polymer is preferably 0.01 to 1% (w/v), more preferably 0.03 to 0.5% (w/v), further preferably 0.05 to 0.3% (w/v),

and most preferably 0.05 to 0.2% (w/v).

As described above, various additives can be added to the present ophthalmic aqueous composition, and when the present active ingredient is rebamipide, it is preferable to add, to the present ophthalmic aqueous composition, an ionic tonicity agent such as sodium chloride or potassium chloride, a buffer such as a sodium citrate hydrate, a pH adjustor and the like in addition to the polyvinylpyrrolidone and the carboxyl vinyl polymer.

When the present active ingredient is sirolimus, the surfactant described above can be added to the present ophthalmic aqueous composition. The surfactant to be added to the present ophthalmic aqueous composition is preferably one or more surfactants selected from the group consisting of a polyoxyethylene fatty acid ester, a polyoxyethylene sorbitan fatty acid ester, polyoxyethylene hardened castor oil, polyoxyl castor oil, polyoxyethylene alkyl ether phosphate, polyoxyl 40 stearate, polysorbate 80, poloxyl 35 castor oil, and sodium polyoxyethylene cetyl ether phosphate, and is particularly preferably polysorbate 80.

When the present active ingredient is sirolimus, a concentration of the surfactant to be added to the present ophthalmic aqueous composition is preferably 0.0001 to 5% (w/v), more preferably 0.001 to 2% (w/v), further preferably 0.001 to 1% (w/v), further preferably 0.002 to 1% (w/v), further preferably 0.005 to 1% (w/v), further preferably 0.005 to 0.5% (w/v), further preferably 0.01 to 1% (w/v), further preferably 0.01 to 0.5% (w/v), and particularly preferably 0.01 to 0.1% (w/v).

Besides, when the present active ingredient is sirolimus, the pH of the present ophthalmic aqueous composition may be within a pharmaceutically acceptable range, and is preferably about 5 from the viewpoint of stability of the present ophthalmic aqueous composition. Specifically, when the present active ingredient is sirolimus, the pH of the present ophthalmic aqueous composition is preferably 4 to 6, more preferably 4.0 to 6.0, further preferably 4.1 to 5.9, further preferably 4.5 to 5.5, further preferably 4.7 to 5.3, and particularly preferably 5.0.

Specifically, when the present active ingredient is sirolimus, the present ophthalmic aqueous composition can be formed as an ophthalmic aqueous composition containing sirolimus, a surfactant, and silver nitrate, having pH of 4 to 6, and filled in a multi-dose eye drop container made of polyethylene.

Besides, an average particle size (D50) of the sirolimus to be contained in the present ophthalmic aqueous composition is 0.001 to 45 μm, preferably 0.001 to 15 μm, more preferably 0.001 to 10 μm, further preferably 0.001 to 8 μm, further preferably 0.001 to 5 μm, further preferably 0.001 to 2.5 μm, further preferably 0.001 to 1 μm, further preferably 0.01 to 0.5 μm, and still further preferably 0.1 to 1 μm. The average particle size is particularly preferably 0.01 to 0.3 μm.

When the present active ingredient is sirolimus, a dispersant can be further added to the present ophthalmic aqueous composition. Examples of the dispersant include cellulose-based polymers such as methylcellulose, ethylcellulose, hydroxymethylcellulose, hydroxyethylcellulose, hydroxypropylcellulose, hydroxyethyl methylcellulose, hydroxypropyl methylcellulose, carboxymethylcellulose, sodium carboxymethylcellulose, hydroxypropyl methylcellulose acetate succinate, hydroxypropyl methylcellulose phthalate, carboxymethylethylcellulose, and cellulose acetate phthalate; polyvinylpyrrolidone; polyhydric alcohols such as polyvinyl alcohol, and polyethylene glycol; carboxyvinyl polymers; and mucopolysaccharides such as sodium hyaluronate, and chondroitin sulfate, and the dispersant may be a hydrate or a solvate thereof.

As described above, various additives can be added to the present ophthalmic aqueous composition, and when the present active ingredient is sirolimus, it is preferable to add, to the present ophthalmic aqueous composition, an ionic tonicity agent such as sodium chloride or potassium chloride, a stabilizer such as a disodium edetate hydrate, a buffer such as a sodium citrate hydrate, a pH adjustor and the like in addition to the surfactant and the dispersant.

The present ophthalmic aqueous composition can be also formed as an ophthalmic aqueous composition containing the active ingredient (excluding sirolimus or a salt thereof), and a silver salt, and filled in a container made of a polyester-based resin, or a container made of a polyolefin-based resin excluding polypropylene.

The definitions of the terms, the preferable examples, the preferable numerical ranges and the like described above regarding the present ophthalmic aqueous composition are also applied to a present ophthalmic preservative and a present method.

Besides, in the present method, the term "imparting preservative efficacy satisfying a criterion of Preservatives-Effectiveness Tests of The Japanese Pharmacopoeia" means that a target composition has preservative efficacy satisfying the criterion of the Preservatives-Effectiveness Tests of the Japanese Pharmacopoeia as a result of subjecting the composition to a test in accordance with the Preservatives-Effectiveness Tests method of the 17th edition of the Japanese Pharmacopoeia.

Now, results of tests performed using the present ophthalmic aqueous composition and preparation examples thereof will be described, and it is noted that these examples are given for further understanding of the present invention and do not limit the scope of the present invention.

Examples

[Test 1]

Silver nitrate was added to a diquafosol sodium-containing aqueous solution in various concentrations, and preservative efficacy of the resultant aqueous solutions was examined.

(Sample Preparation Method)

Formulation 1-1: In accordance with a prescription shown in Table 1, a formulation 1-1 was prepared. Specifically, diquafosol sodium (3 g), silver nitrate (0.00008 g), a sodium hydrogen phosphate hydrate (0.2 g), a disodium edetate hydrate (0.01 g), polyvinylpyrrolidone K30 (PVP K30) (2 g), concentrated glycerin (1.2 g), and hydroxyethylcellulose (0.25 g) were dissolved in sterile purified water to a volume of 100 mL, and a pH adjustor was added thereto to pH 7.5.

Formulations 1-2 to 1-5: In accordance with prescriptions shown in Table 1, formulations 1-2 to 1-5 were prepared in the same manner as formulation 1-1.

[Table 1]

| (unit: g/100 mL in Table 1) | | | | | |
|---|---|---|---|---|---|
| Component | Formulation 1-1 | Formulation 1-2 | Formulation 1-3 | Formulation 1-4 | Formulation 1-5 |
| Diquafosol Sodium | 3 | 3 | 3 | 3 | 3 |
| Silver Nitrate | 0.00008 | 0.00004 | 0.000016 | 0.000008 | 0.000004 |
| Sodium Hydrogen Phosphate Hydrate | 0.2 | 0.2 | 0.2 | 0.2 | 0.2 |
| Disodium Edetate Hydrate | 0.01 | 0.01 | 0.01 | 0.01 | 0.01 |
| PVP K30 | 2 | 2 | 2 | 2 | 2 |
| Sodium Chloride | - | 0.45 | 0.45 | 0.45 | 0.45 |
| Concentrated Glycerin | 1.2 | - | - | - | - |
| Hydroxyethylcellulose | 0.25 | 0.25 | 0.25 | 0.25 | 0.25 |
| pH Adjustor | q.s. | q.s. | q.s. | q.s. | q.s. |
| pH | 7.5 | 7.5 | 7.5 | 7.5 | 7.5 |

(Test Method)

Preservatives-Effectiveness Tests was performed in accordance with the Preservatives-Effectiveness Tests method of the 17th edition of the Japanese Pharmacopoeia. In the present test, *Escherichia Coli* (*E. coli*), *Pseudomonas aeruginosa* (*P. aeruginosa*), *Staphylococcus aureus* (*S. aureus*), *Candida albicans* (*C. albicans*) and *Aspergillus brasiliensis* (*A. brasiliensis*) were used as test bacteria.

(Results)

Test results are shown in Table 2. It was revealed that formulations 1-1 to 1-5 satisfy the criterion of the Preservatives-Effectiveness Tests of The Japanese Pharmacopoeia. It is noted that the test results shown in Table 2 indicate, as log reduction, how much a viable bacterial count was reduced as compared with the inoculated bacterial count in the test, and for example, a test result "1" indicates that the viable bacterial count was reduced to 10% of the inoculated bacterial count in the test.

[Table 2]

| ( Value: Log Reduction) | | | | | | |
|---|---|---|---|---|---|---|
| | | Formulation 1-1 | Formulation 1-2 | Formulation 1-3 | Formulation 14 | Formulation 1-5 |
| E.coli | 1 week | 4.6 | 4.7 | 4.7 | 4.7 | 4.7 |
| | 2 weeks | 4.6 | 4.7 | 4.7 | 4.7 | 4.7 |
| | 4 weeks | 4.6 | 4.7 | 4.7 | 4.7 | 4.7 |
| P.aeruginosa | 1 week | 4.7 | 4.7 | 4.7 | 4.7 | 4.7 |
| | 2 weeks | 4.7 | 4.7 | 4.7 | 4.7 | 4.7 |
| | 4 weeks | 4.7 | 4.7 | 4.7 | 4.7 | 4.7 |
| S.aureus | 1 week | 4.7 | 4.6 | 3.1 | 2.3 | 1.8 |
| | 2 weeks | 4.7 | 4.6 | 4.6 | 4.6 | 4.6 |
| | 4 weeks | 4.7 | 4.6 | 4.6 | 4.6 | 4.6 |
| C.albicans | 1 week | 4.3 | 3.9 | 3.1 | 2.6 | 2.0 |
| | 2 weeks | 4.6 | 4.8 | 4.8 | 4.8 | 4.2 |
| | 4 weeks | 4.6 | 4.8 | 4.8 | 4.8 | 4.8 |
| A.brasiliensis | 1 week | 3.2 | 0.5 | 0.2 | 0.2 | 0.2 |
| | 2 weeks | 4.3 | 0.7 | 0.3 | 0.2 | 0.2 |
| | 4 weeks | 4.3 | 1.1 | 0.4 | 0.4 | 0.3 |
| Determination | | Satisfy | Satisfy | Satisfy | Satisfy | Satisfy |

(Discussion)

It was revealed that a silver salt such as silver nitrate can be a novel preservative replaceable with existing preservatives such as benzalkonium chloride and chlorhexidine gluconate in preparation of an aqueous ophthalmic solution.

[Test 2]

Silver nitrate was added to a rebamipide-containing aqueous suspension in various concentrations, and preservative efficacy of the resultant suspensions was examined. Besides, it was also examined whether or not the concentration of the silver nitrate in each of the suspensions was varied during preservation.

(Sample Preparation Method)

Formulation 2-1: In accordance with a prescription shown in Table 3, a formulation 2-1 was prepared. Specifically, 0.146 g of a sodium citrate hydrate, 0.65 g of sodium chloride, 0.18 g of potassium chloride, 2 g of polyvinylpyrrolidone K30, 0.11 g of a carboxyvinyl polymer (CARBOPOL (registered trademark) 971PNF), and 0.00004 g of silver nitrate were dissolved in water, 2.0 g of rebamipide was added to the resultant to be suspended by stirring, the resultant was adjusted to pH 5.9, and water was added thereto to a volume of 100 mL.

Formulations 2-2 to 2-3: In accordance with prescriptions shown in Table 3, these formulations were prepared in the same manner as formulation 2-1.

[Table 3]

| ( unit: g/100 mL in Table 3) | | | |
|---|---|---|---|
| Component | Formulation 2-1 | Formulation 2-2 | Formulation 2-3 |
| Rebamipide | 2 | 2 | 2 |
| Partially Saponified Product of Polyvinyl Alcohol | - | - | - |

(continued)

| ( unit: g/100 mL in Table 3) | | | |
|---|---|---|---|
| Component | Formulation 2-1 | Formulation 2-2 | Formulation 2-3 |
| Polyvinylpyrrolidone K30 | 2 | 2 | 2 |
| Carboxyvinyl Polymer | 0.11 | 0.11 | 0.11 |
| Silver Nitrate | 0.00004 | 0.00003 | 0.00002 |
| Sodium Citrate Hydrate | 0.146 | 0.146 | 0.146 |
| Sodium Chloride | 0.65 | 0.65 | 0.65 |
| Potassium Chloride | 0.18 | 0.18 | 0.18 |
| pH | 5.9 | 5.9 | 5.9 |

(Test Method)

<Preservatives-Effectiveness Tests>

Preservatives-Effectiveness Tests was performed in accordance with the Preservatives-Effectiveness Tests method of the 17th edition of the Japanese Pharmacopoeia. In the present test, *Escherichia Coli* (*E. coli*), *Pseudomonas aeruginosa* (*P. aeruginosa*), *Staphylococcus aureus* (*S. aureus*), *Candida albicans* (*C. albicans*) and *Aspergillus brasiliensis* (*A. brasiliensis*) were used as test bacteria.

<Stability Test>

Each of formulation 2-1 and formulation 2-3 was put in an eye drop container made of low density polyethylene (LDPE) in an amount of 5 mL each, and the resultant was preserved at 40°C for 3 months, or under exposure to light of 1.2 million lx/hr. A content of the silver nitrate in each medicinal solution was measured before and after the preservation by "Inductively Coupled Plasma-Mass Spectrometry of the Japanese Pharmacopoeia".

(Test Results)

Test results are shown in Table 4 and Table 5. It was revealed that formulations 2-1 to 2-3 satisfy the criterion of the Preservatives-Effectiveness Tests of The Japanese Pharmacopoeia. Besides, the concentration of the silver nitrate in each of the rebamipide-containing aqueous suspensions was not varied during the preservation.

[Table 4]

| ( Value: Log Reduction) | | | | |
|---|---|---|---|---|
| | | Formulation 2-1 | Formulation 2-2 | Formulation 2-3 |
| E.coli | 1 week | 4.5 | 4.5 | 4.5 |
| | 2 weeks | 4.5 | 4.5 | 4.5 |
| | 4 weeks | 4.5 | 4.5 | 4.5 |
| P.aeruginosa | 1 week | 4.6 | 4.6 | 4.6 |
| | 2 weeks | 4.6 | 4.6 | 4.6 |
| | 4 weeks | 4.6 | 4.6 | 4.6 |
| S.aureus | 1 week | 4.7 | 4.7 | 4.7 |
| | 2 weeks | 4.7 | 4.7 | 4.7 |
| | 4 weeks | 4.7 | 4.7 | 4.7 |

(continued)

| ( Value: Log Reduction) | | | | |
|---|---|---|---|---|
| | | Formulation 2-1 | Formulation 2-2 | Formulation 2-3 |
| C.albicans | 1 week | 0.1 | 0.0 | -0.1 |
| | 2 weeks | 0.1 | -0.1 | -0.2 |
| | 4 weeks | 0.0 | -0.1 | -0.1 |
| Abrasiliensis | 1 week | -0.1 | -0.1 | -0.03 |
| | 2 weeks | -0.1 | 0.0 | -0.1 |
| | 4 weeks | -0.1 | 0.0 | 0.0 |
| Determination | | Satisfy | Satisfy | Satisfy |

[Table 5]

| Name of Sample | Ag Concentration(μg/L) |
|---|---|
| Formulation2-1_initial | 220 |
| Formulation2-1_ light of 1.2 million l×/hr | 210 |
| Formulation2-1 40°C3M | 220 |
| Formulation2-3_initial | 120 |
| Formulation2-3_ light of 1.2 million l×/hr | 100 |
| Formulation2-3_ 40°C3M | 110 |

(Discussion)

It was revealed that a silver salt such as silver nitrate can be a novel preservative replaceable with existing preservatives such as benzalkonium chloride and chlorhexidine gluconate also in an aqueous ophthalmic suspension. In other words, it was revealed that a silver salt such as silver nitrate can be usable as a preservative in both an aqueous ophthalmic solution and an aqueous ophthalmic suspension.

Besides, it was revealed that a silver salt such as silver nitrate is stable in a rebamipide-containing aqueous suspension.

[Test 3]

Stability of silver nitrate and chlorhexidine gluconate in a diquafosol sodium-containing aqueous ophthalmic solution was compared.

(Sample Preparation Method)

Formulation 3-1: In accordance with a prescription shown in Table 3, a formulation 3-1 was prepared. Specifically, diquafosol sodium (3 g), silver nitrate (0.00008 g), PVP K30 (2 g), hydroxyethylcellulose (0.25 g), a sodium hydrogen phosphate hydrate (0.2 g), a disodium edetate hydrate (0.01 g), and sodium chloride (0.45 g) were dissolved in water to a volume of 100 mL, and a pH adjustor (q.s.) was added thereto to pH 7.5.

Comparative Formulations 3-1 to 3-4: In accordance with prescriptions shown in Table 6, comparative formulations 3-1 to 3-4 were prepared in the same manner as formulation 3-1.

[Table 6]

| ( unit: gI100 mL in Table 6) | | | | | |
|---|---|---|---|---|---|
| Component | Formulation 3-1 | Comparative Formulation | | | |
| | | 3-1 | 3-2 | 3-3 | 3-4 |
| Diquafosol Sodium | 3 | 3 | 3 | 3 | 3 |
| Silver Nitrate (preservative) | 0.00008 | - | - | - | - |
| Chlorhexidine Gluconate (preservative) | - | 0.0025 | 0.0025 | 0.0025 | 0.0025 |
| PVP K30 | 2 | - | 2 | - | - |
| PVP K90 | - | - | - | 2 | 4 |
| Hydroxyethylcellulose | 0.25 | 0.2 | 0.2 | - | - |
| Sodium Hydrogen Phosphate Hydrate | 0.2 | 0.2 | 0.2 | 0.2 | 0.2 |
| Disodium Edetate Hydrate | 0.01 | 0.01 | 0.01 | 0.01 | 0.01 |
| Sodium Chloride | 0.45 | 0.45 | 0.45 | - | - |
| Concentrated Glycerin | - | - | - | 1.2 | 1.2 |
| pH Adjustor | q.s. | q.s. | q.s. | q.s. | q. s. |
| pH | 7.5 | 7.5 | 7.5 | 7.5 | 7.5 |

(Test Method)

A silver ion content in formulation 3-1 having been preserved at 60°C for 4 weeks was quantitatively determined by high-frequency Inductively Coupled Plasma-Atomic Emission Spectroscopy (ICP-AES) to calculate the residual rate (%). Besides, a chlorhexidine gluconate content in each of comparative formulations 3-1 and 3-2 having been preserved at 60°C for 4 weeks was quantitatively determined by high performance liquid chromatography (HPLC) to calculate the residual rate (%). Besides, a chlorhexidine gluconate content in each of comparative formulations 3-3 and 3-4 having been preserved at 60°C for 2 weeks was quantitatively determined by high performance liquid chromatography (HPLC) to calculate the residual rate (%).

(Test Results)

Test results are shown in Table 7. In formulation 3-1 containing diquafosol sodium as the active ingredient, and PVP K30 and the like as the additives, the silver ion content was not changed. On the contrary, in comparative formulations 3-2 to 3-4 containing diquafosol sodium as the active ingredient, and PVP K30 or PVP K90 and the like as the additives, the chlorhexidine gluconate content was reduced.

[Table 7]

| | Formulation 3-1 | Comparative Formulation | | | |
|---|---|---|---|---|---|
| | | 3-1 | 3-2 | 3-3 | 3-4 |
| Residual Rate (%) of Silver Ion or Chlorhexidine Gluconate | 100 | 90.1 | 20.4 | 26.7 | 8.0 |

(Discussion)

As described above in BACKGROUND ART, chlorhexidine gluconate is used in an ophthalmic aqueous composition as a preservative safer than benzalkonium chloride, but was thus revealed to be unstabilized in a composition depending on types of an active ingredient and an additive to be used. On the contrary, a silver salt such as silver nitrate exhibited high stability in a diquafosol sodium-containing aqueous ophthalmic solution, and hence was thus revealed to be a preservative widely usable and stable without depending on an active ingredient and an additive to be used, and properties of the ophthalmic solution.

[Test 4]

Stability of chlorhexidine gluconate in a rebamipide-containing aqueous ophthalmic solution was examined.

(Sample Preparation Method)

Comparative Formulation 4-1: Commercially available "Mucosta (registered trademark) ophthalmic suspension UD 2%" was used.

Comparative Formulation 4-2: In accordance with a prescription shown in Table 8, a comparative formulation 4-2 was prepared. Specifically, 0.15 g of a sodium citrate hydrate, 0.72 g of sodium chloride, 0.18 g of potassium chloride, 1 g of a partially saponified product of polyvinyl alcohol, and 0.01 g of chlorhexidine gluconate were dissolved in water, 2 g of rebamipide was added to the resultant to be suspended by stirring, the resultant was adjusted to pH 6.0, and water was added thereto to a volume of 100 mL.

Comparative Formulation 4-3: In accordance with a prescription shown in Table 8, this formulation was prepared in the same manner as comparative formulation 4-2.

(Test Method)

Preservatives-Effectiveness Tests was performed in accordance with the Preservatives-Effectiveness Tests method of the 17th edition of the Japanese Pharmacopoeia. In the present test, all of or some of *Escherichia Coli* (*E. coli*), *Pseudomonas aeruginosa* (*P. aeruginosa*), *Staphylococcus aureus* (*S. aureus*), *Candida albicans* (*C. albicans*) and *Aspergillus brasiliensis* (*A. brasiliensis*) were used as test bacteria. It is noted that a chlorhexidine gluconate content was measured by "high performance chromatography of the Japanese Pharmacopoeia".

(Results)

Measurement results are shown in Table 8. Comparative formulations 4-1 to 4-3 did not satisfy the Preservatives-Effectiveness Tests.

[Table 8]

| ( unit: g/100 mL in Table 8) | | | | | |
|---|---|---|---|---|---|
| Component | | | Comparative Formulation 4-1 | Comparative Formulation 4-2 | Comparative Formulation 4-3 |
| Rebamipide | | | 2 | 2 | 2 |
| Partially Saponified Product of Polyvinyl Alcohol | | | 1 | 1 | 1 |
| Chlorhexidine Gluconate | | | - | 0.01 | 0.005 |
| Sodium Citrate Hydrate | | | 0.15 | 0.15 | 0.15 |
| Sodium Chloride | | | 0.72 | 0.72 | 0.72 |
| Potassium Chloride | | | 0.18 | 0.18 | 0.18 |
| pH | | | 6.0 | 6.0 | 6.0 |
| Osmotic Pressure Ratio | | | 0.9-1.1 | 0.9-1.1 | 0.9-1.1 |
| Amount of Chlorhexidine Gluconate against Display Amount | | | - | 3.55% | 3.71% |
| Amount of Chlorhexidine Gluconate against Display Amount with Rebamipide dissolved | | | - | 98.57% | 103.74% |
| Bacterium | Preservation Period | Criterion | Logarithmic Reduction | | |
| *E.coli* | 7d | 1.0 or more | -0.2 | -0.4 | -0.4 |
| | 14d | 3.0 or more | 0.0 | -0.3 | -0.4 |
| | 28d | N.I.* | 0.6 | -0.1 | -0.2 |

(continued)

| Bacterium | Preservation Period | Criterion | Logarithmic Reduction | | |
|---|---|---|---|---|---|
| *P.aeruginosa* | 7d | 1.0 or more | 0.2 | -0.1 | -0.2 |
| | 14d | 3.0 or more | 0.1 | -0.4 | -0.5 |
| | 28d | N.I.* | -0.1 | -0.4 | -0.5 |
| *S.aureus* | 7d | 1.0 or more | -0.1 | 0.3 | 0.3 |
| | 14d | 3.0 or more | 3.9 | 2.1 | 2.4 |
| | 28d | N.I.* | >4.6 | >4.6 | >4.6 |
| *C.albicans* | 7d | N.I.* | 0.4 | 0.6 | 0.5 |
| | 14d | N.I.* | 0.1 | -0.2 | -0.2 |
| | 28d | N.I.* | -0.1 | -0.1 | -0.2 |
| *A.brasiliensis* | 7d | N.I.* | 0.2 | 0.1 | 0.4 |
| | 14d | N.I.* | 0.1 | 0.2 | -0.1 |
| | 28d | N.I.* | 0.3 | 0.2 | 0.1 |
| *N.I.:Not Increase | | | | | |

(Discussion)

As described above in BACKGROUND ART, chlorhexidine gluconate is used in an ophthalmic aqueous composition as a preservative safer than benzalkonium chloride, but it was thus suggested that compound change may be caused and sufficient preservative efficacy cannot be exhibited depending on types of an active ingredient and an additive to be used. On the contrary, as described in Test 2, a silver salt such as silver nitrate did not cause compound change and the like in a rebamipide-containing aqueous ophthalmic solution, and hence was revealed to be a preservative widely usable and stable without depending on an active ingredient and an additive to be used, and properties of the ophthalmic solution.

[Test 5]

Influence of silver nitrate on a soft contact lens (SCL) was examined.

(Sample Preparation Method)

Formulation 5-1: In accordance with a prescription shown in Table 9, a formulation 5-1 was prepared. Specifically, silver nitrate (0.0004 g), a sodium hydrogen phosphate hydrate (0.2 g), and sodium chloride (0.9 g) were dissolved in water to a volume of 100 mL, and a pH adjustor (q.s.) was added thereto to pH 7.0.

Comparative Formulations 5-1 to 5-4: In accordance with prescriptions shown in Table 9, comparative formulations 5-1 to 5-4 were prepared in the same manner as formulation 5-1.

[Table 9]

| | Formulation 5-1 | Comparative Formulation | | | |
|---|---|---|---|---|---|
| | | 5-1 | 5-2 | 5-3 | 54 |
| Sodium Hydrogen Phosphate Hydrate | 0.2 | 0.2 | 0.2 | 0.2 | 0.2 |
| Sodium Chloride | 0.9 | 0.9 | 0.9 | 0.9 | 0.9 |
| Silver Nitrate | 0.0004 | - | - | - | - |
| Polyhexanide Hydrochloride | - | - | 0.01 | - | - |
| Benzalkonium Chloride | - | - | - | 0.01 | - |

(continued)

| | Formulation 5-1 | Comparative Formulation | | | |
|---|---|---|---|---|---|
| | | 5-1 | 5-2 | 5-3 | 54 |
| Chlorhexidine Gluconate | - | - | - | - | 0.01 |
| pH Adjustor | q.s. | q.s. | q.s. | q.s. | q.s. |
| pH | 7.0 | 7.0 | 7.0 | 7.0 | 7.0 |

(Test Method)

Soft contact lenses were immersed in the test preparations at room temperature for 30 minutes, and then taken out. The diameter and the base curve of each of the soft contact lenses were measured. It is noted that the soft contact lenses used herein were Two-week Acuvue (registered trademark) (Johnson & Johnson) classified as Group IV.

It is noted that diameter deformation and base curve deformation were calculated in accordance with the following expression:

Diameter deformation (mm) = (diameter after immersion) - (diameter before immersion)

Base curve deformation (mm) = (base curve after immersion) - (base curve before immersion)

(Test Results)

Test results are shown in Table 10.

[Table 10]

| | Formulation 5-1 | Comparative Formulation | | | |
|---|---|---|---|---|---|
| | | 5-1 | 5-2 | 5-3 | 5-4 |
| Diameter Deformation (mm) | -0.02 | 000 | -0.19 | -0.22 | -0.17 |
| Base Curve Deformation (mm) | 0.01 | 000 | 0.06 | -0.08 | 0.00 |

(Discussion)

Benzalkonium chloride, which is known to deform a SCL, was found, also in the present test, to be liable to deform a SCL as compared with other preservatives. On the contrary, a silver nitrate-containing formulation substantially did not deform a SCL, and this trend was conspicuous even as compared with chlorhexidine gluconate-containing formulation, which is deemed not to deform a SCL. Accordingly, it was revealed that an ophthalmic aqueous composition containing a silver salt such as silver nitrate as a preservative does not deform a SCL, and can be instilled into an SCL wearing eye.

[Test 6]

Adsorption of a silver ion onto various resins was examined.

(Sample Preparation Method)

Formulation 6-1: An aqueous solution containing 10 ppm of a silver ion manufactured by and commercially available from Japan Ion Corporation was diluted with pure water to 3 ppm (0.0003% (w/v)) to prepare a sample.

(Test Method)

Formulation 6-1 was filled in an amount of 5 mL each in an eye drop container made of low density polyethylene (LDPE), an eye drop container made of polypropylene (PP), and an eye drop container made of polyethylene terephthalate (PET), and a silver ion content in each of these containers immediately after the filling and after preservation at 60°C for 4 weeks was quantitatively determined by high-frequency Inductively Coupled Plasma-Atomic Emission Spectroscopy (ICP-AES) to calculate a residual rate (%) after the preservation against the content immediately after the filling.

(Test Results)

Test results are shown in Table 11. It was revealed that a silver ion strongly adsorbs onto polypropylene.

[Table 11]

| | LDPE Container | PP Container | PET Container |
|---|---|---|---|
| Residual Rate | 89% | 0%(N.D.) | 110% |

(Discussion)

Although an ophthalmic aqueous composition is filled in a resin container in general, it was revealed that an ophthalmic composition containing a silver salt such as silver nitrate is preferably filled in a resin container excluding a polypropylene container for avoiding deterioration of the preservative efficacy through the adsorption of a silver ion.

[Test 7]

Adsorption of a silver ion onto a resin in a diquafosol sodium-containing aqueous solution was examined.

(Sample Preparation Method)

Formulation 7-1: In accordance with a prescription shown in Table 12, a formulation 7-1 was prepared. Specifically, diquafosol sodium (3 g), silver nitrate (0.00004 g), PVP K30 (2 g), hydroxyethylcellulose (0.25 g), a sodium hydrogen phosphate hydrate (0.2 g), a disodium edetate hydrate (0.01 g), and sodium chloride (0.45 g) were dissolved in water to a volume of 100 mL, and a pH adjustor (q.s.) was added thereto to pH 7.5.

Formulation 7-2: In accordance with a prescription shown in Table 12, a formulation 7-2 was prepared in the same manner as formulation 7-1.

[Table 12]

| ( unit: g/100 mL in Table 12) | | |
|---|---|---|
| Component | Formulation 7-1 | Formulation 7-2 |
| Diquafosol Sodium | 3 | 3 |
| Silver Nitrate (preservative) | 0.00004 | 0.00004 |
| PVP K30 | 2 | 2 |
| Hydroxyethylcellulose | 0.25 | 0.25 |
| Sodium Hydrogen Phosphate Hydrate | 0.2 | 0.2 |
| Disodium Edetate Hydrate | 0.01 | 0.01 |
| Sodium Chloride | 0.45 | - |
| D-mannitol | - | 2.2 |
| pH Adjustor | q.s. | q.s. |
| pH | 7.5 | 7.5 |

(Test Method)

Each of formulation 7-1 and formulation 7-2 was filled in an eye drop container made of low density polyethylene (LDPE), and preserved at 60°C for 4 weeks, and then a silver content in the resultant was quantitatively determined by high-frequency Inductively Coupled Plasma-Atomic Emission Spectroscopy (ICP-AES) to calculate a residual rate (%) after the preservation against the content immediately after the filling.

(Test Results)

Test results are shown in Table 13. It was confirmed that a silver ion adsorbs onto the eye drop container made of low density polyethylene (LDPE) in formulation 7-2, but the adsorption was completely suppressed by adding sodium chloride thereto.

[Table 13]

| | Formulation 7-1 | Formulation 7-2 |
|---|---|---|
| Residual Rate | 102% | 9% |

(Discussion)

In the present test, the concentration of the silver nitrate added to the aqueous composition was 0.00004% (w/v), which was a rather low concentration as compared with the concentration employed in Test 6 (0.003% (w/v)), and this was probably because negligible adsorption of a silver ion onto the eye drop container made of low density polyethylene (LDPE) was caused. On the contrary, it was suggested that the adsorption of a silver ion onto a resin container can be remarkably suppressed by adding an ionic tonicity agent such as sodium chloride.

[Test 8]

Silver nitrate was added to a sirolimus-containing aqueous suspension in various concentrations, and preservative efficacy of the resultant suspensions was examined. Besides, it was also examined whether or not the concentration of the silver nitrate in each of the suspensions was varied during preservation.

(Sample Preparation Method)

Comparative Formulation 8-1: In accordance with a prescription shown in Table 14, a comparative formulation 8-1 was prepared. Specifically, sirolimus, polysorbate 80 and purified water were mixed, the resultant was subjected to wet grinding with a bead mill, an additive solution shown in Table 14 was then added to the resultant to be mixed, and a pH adjustor was added thereto to pH 5.

Formulation 8-1 to 8-2: In accordance with prescriptions shown in Table 14, these formulations were prepared in the same manner as comparative formulation 8-1.

[Table 14]

| (unit: g/100 mL in Table 14) | | | |
|---|---|---|---|
| | Comparative Formulation 8-1 | Formulation 8-1 | Formulation 8-2 |
| Sirolimus | 0.05 | 0.05 | 0.05 |
| Silver Nitrate | - | 0.00002 | 0.00004 |
| (Concentration in terms of silver) | - | (127μg/L) | (254μg/L) |
| Polysorbate 80(W) | 0.05 | 0.05 | 0.05 |
| Hypromellose (TC-5R) | 0.0002 | 0.0002 | 0.0002 |
| Sodium Chloride | 0.8 | 0.8 | 0.8 |

(continued)

| (unit: g/100 mL in Table 14) | | | |
|---|---|---|---|
| | Comparative Formulation 8-1 | Formulation 8-1 | Formulation 8-2 |
| Sodium Citrate Hydrate | 0.05 | 0.05 | 0.05 |
| Sodium Dihydrogen Phosphate Hydrate | 0.05 | 0.05 | 0.05 |
| Disodium Edetate Hydrate | 0.005 | 0.005 | 0.005 |
| Sodium Hydroxide/Dilute Hydrochloric Acid | q.s. | q.s. | q.s. |
| Purified Water | q.s. | q.s. | q.s. |
| pH | 5 (4-6) | 5 (4-6) | 5 (4-6) |

(Test Method)

<Preservatives-Effectiveness Tests>

Preservatives-Effectiveness Tests was performed in accordance with the Preservatives-Effectiveness Tests method of the 17th edition of the Japanese Pharmacopoeia. In the present test, *Escherichia Coli* (*E. coli*), *Pseudomonas aeruginosa* (*P. aeruginosa*), *Staphylococcus aureus* (*S. aureus*), *Candida albicans* (*C. albicans*) and *Aspergillus brasiliensis* (*A. brasiliensis*) were used as test bacteria.

<Stability Test>

Each of comparative formulation 8-1 and formulations 8-1 to 8-2 was put in an eye drop container made of low density polyethylene (LDPE) in an amount of 5 mL, and the resultant was preserved at 60°C for 4 weeks. A content of the silver nitrate in each medicinal solution was measured before and after the preservation by "Inductively Coupled Plasma-Mass Spectrometry of the Japanese Pharmacopoeia".

(Test Results)

Test results are shown in Table 15 and Table 16. It was revealed that formulation 8-1 and formulation 8-2 satisfy the criterion of the Preservatives-Effectiveness Tests of The Japanese Pharmacopoeia. Besides, the concentration of the silver nitrate in each of the sirolimus-containing aqueous suspensions was not varied during the preservation.

[Table 15]

| Bacterium | Preservation Period | Criterion | Comparative Formulation 8-1 | Formulation 8-1 | Formulation 8-2 |
|---|---|---|---|---|---|
| *E.coli* | 7d | 1.0 | 0 | 1.7 | 3.8 |
| | 14d | 3.0 | 0.1 | 3.0 | >4.7 |
| | 28d | N.I.’ | 0.5 | >4.7 | >4.7 |
| *P.aeruginosa* | 7d | 1.0 | 1.2 | 4.3 | >4.6 |
| | 14d | 3.0 | 1.5 | >4.6 | >4.6 |
| | 28d | N.I.’ | 2.9 | >4.6 | >4.6 |
| *S.aureus* | 7d | 1.0 | 2.6 | >4.8 | >4.8 |
| | 14d | 3.0 | >4.8 | >4.8 | >4.8 |
| | 28d | N.I.’ | >4.8 | >4.8 | >4.8 |
| *C.albicans* | 7d | N.I.’ | 1.1 | 1.1 | 1.4 |
| | 14d | N.I.’ | 3.1 | 2.7 | 3.2 |
| | 28d | N.I.’ | >4.6 | >4.6 | >4.6 |

(continued)

| Bacterium | Preservation Period | Criterion | Comparative Formulation 8-1 | Formulation 8-1 | Formulation 8-2 |
|---|---|---|---|---|---|
| *A.brasiliensis* | 7d | N.I.' | 0.1 | 0.3 | 0.3 |
| | 14d | N.I.' | 0.3 | 0.3 | 0.3 |
| | 28d | N.I.' | 0.3 | 0.3 | 0.3 |
| *N.I.:Not Increase | | | | | |

[Table 16]

| Name of Sample | Ag Concentration(μg/L) |
|---|---|
| Formulation8-1 Initial | 142 |
| Formulation8-1 after preservation at 60°C for 4 weeks | 128 |
| Formulation8-2 Initial | 264 |
| Formulation8-2 after preservation at 60°C for 4 weeks | 255 |

(Discussion)

It was revealed that a silver salt such as silver nitrate can be a novel preservative replaceable with existing preservatives such as benzalkonium chloride and chlorhexidine gluconate also in a sirolimus-containing aqueous ophthalmic suspension. Although the sirolimus-containing aqueous ophthalmic suspension contains a surfactant differently from the diquafosol sodium-containing aqueous ophthalmic solution and the rebamipide-containing aqueous ophthalmic suspension described above, it was revealed that sufficient preservative efficacy can be obtained by containing silver nitrate in a concentration of 0.00002% (w/v) or more. In other words, it was revealed that a silver salt such as silver nitrate can be usable as a preservative in both an aqueous ophthalmic solution and an aqueous ophthalmic suspension regardless of types of an active ingredient and an additive.

[Preparation Examples]

The agent of the present invention will now be more specifically described with reference to preparation examples, and it is noted that the present invention is not limited only to these preparation examples.

(Preparation Example 1) in 100 mL

| | |
|---|---|
| diquafosol sodium | 3 g |
| sodium hydrogen phosphate hydrate | 0.01 to 0.5 g |
| sodium chloride | 0.01 to 0.9 g |
| disodium edetate hydrate | 0.0001 to 0.1 g |
| polyvinylpyrrolidone K90 | 0.0001 to 10 g |
| silver nitrate | 0.0000001 to 0.01 g |
| pH adjustor | q.s. |
| purified water | q.s. |
| pH | 7.0 to 8.0 |

This preparation is filled in a multi-dose eye drop container made of low density polyethylene (LDPE).

(Preparation Example 2) in 100 mL

| | |
|---|---|
| rebamipide | 3 g |
| sodium citrate hydrate | 0.01 to 0.5 g |
| sodium chloride | 0.01 to 0.9 g |
| potassium chloride | 0.01 to 0.9 g |
| polyvinylpyrrolidone K30 | 0.1 to 2 g |
| silver nitrate | 0.0000001 to 0.01 g |
| pH adjuster | q.s. |
| purified water | q.s. |
| pH | 5.5 to 6.5 |

This preparation is filled in a multi-dose eye drop container made of low density polyethylene (LDPE).

(Preparation Example 3) in 100 mL

| | |
|---|---|
| sodium chloride | 0.01 to 0.9 g |
| potassium chloride | 0.01 to 0.9 g |
| silver nitrate | 0.0000001 to 0.01 g |
| pH adjustor | q.s. |
| purified water | q.s. |
| pH | 7.8 to 8.0 |

This preparation is filled in a multi-dose eye drop container made of polyethylene terephthalate.

(Preparation Example 4) in 100 mL

| | |
|---|---|
| sirolimus | 0.01 to 0.1 g |
| polysorbate 80 | 0.005 to 0.1 g |
| hydroxypropyl methylcellulose | 0.0001 to 0.01 g |
| sodium citrate hydrate | 0.05 to 0.1 g |
| disodium edetate hydrate | 0.01 to 0.075 g |
| concentrated glycerin | 1.2 to 2.0 g |
| silver nitrate | 0.00002 to 0.01 g |
| pH adjustor | q.s. |
| purified water | q.s. |
| pH | 5.0 |

This preparation is filled in a multi-dose eye drop container made of low density polyethylene (LDPE).

(Preparation Example 5) in 100 mL

| | |
|---|---|
| sirolimus | 0.01 to 0.1 g |
| polysorbate 80 | 0.005 to 0.1 g |
| hydroxypropyl methylcellulose | 0.0001 to 0.01 g |
| sodium citrate hydrate | 0.05 to 0.1 g |
| disodium edetate hydrate | 0.01 to 0.075 g |
| sodium chloride | 0.7 to 1.2 g |

(continued)

| | |
|---|---|
| silver nitrate | 0.00002 to 0.01 g |
| pH adjustor | q.s. |
| purified water | q.s. |
| pH | 5.0 |

This preparation is filled in a multi-dose eye drop container made of low density polyethylene (LDPE).

(Preparation Example 6) in 100 mL

| | |
|---|---|
| sirolimus | 0.01 to 0.1 g |
| polysorbate 80 | 0.005 to 0.1 g |
| sodium carboxymethylcellulose | 0.001 to 0.01 g |
| sodium citrate hydrate | 0.05 to 0.1 g |
| disodium edetate hydrate | 0.01 to 0.075 g |
| concentrated glycerin | 1.2 to 2.0 g |
| silver nitrate | 0.00002 to 0.01 g |
| pH adjustor | q.s. |
| purified water | q.s. |
| pH | 5.0 |

This preparation is filled in a multi-dose eye drop container made of low density polyethylene (LDPE).

(Preparation Example 7) in 100 mL

| | |
|---|---|
| sirolimus | 0.01 to 0.1 g |
| polysorbate 80 | 0.005 to 0.1 g |
| sodium carboxymethylcellulose | 0.001 to 0.01 g |
| sodium citrate hydrate | 0.05 to 0.1 g |
| disodium edetate hydrate | 0.01 to 0.075 g |
| sodium chloride | 0.7 to 1.2 g |
| silver nitrate | 0.00002 to 0.01 g |
| pH adjustor | q.s. |
| purified water | q.s. |
| pH | 5.0 |

This preparation is filled in a multi-dose eye drop container made of low density polyethylene (LDPE).

(Preparation Example 8) in 100 mL

| | |
|---|---|
| diquafosol sodium | 3 g |
| sodium hydrogen phosphate hydrate | 0.01 to 0.5 g |
| sodium chloride | 0.01 to 0.9 g |
| disodium edetate hydrate | 0.0001 to 0.1 g |
| polyvinylpyrrolidone K60 | 0.0001 to 10 g |
| silver nitrate | 0.0000001 to 0.01 g |
| pH adjustor | q.s. |

(continued)

| | |
|---|---|
| purified water | q.s. |
| pH | 7.0 to 8.0 |

This preparation is filled in a multi-dose eye drop container made of low density polyethylene (LDPE).

(Preparation Example 9) in 100 mL

| | |
|---|---|
| diquafosol sodium | 3 g |
| sodium hydrogen phosphate hydrate | 0.01 to 0.5 g |
| sodium chloride | 0.01 to 0.9 g |
| disodium edetate hydrate | 0.0001 to 0.1 g |
| polyvinylpyrrolidone K30 | 0.0001 to 10 g |
| silver nitrate | 0.0000001 to 0.01 g |
| pH adjustor | q.s. |
| purified water | q.s. |
| pH | 7.0 to 8.0 |

This preparation is filled in a multi-dose eye drop container made of low density polyethylene (LDPE).

(Preparation Example 10) in 100 mL

| | |
|---|---|
| diquafosol sodium | 3 g |
| sodium hydrogen phosphate hydrate | 0.01 to 0.5 g |
| sodium chloride | 0.01 to 0.9 g |
| disodium edetate hydrate | 0.0001 to 0.1 g |
| polyvinylpyrrolidone K90 | 0.0001 to 10 g |
| silver nitrate | 0.0000001 to 0.01 g |
| hydroxyethylcellulose | 0.0001 to 5 g |
| pH adjustor | q.s. |
| purified water | q.s. |
| pH | 7.0 to 8.0 |

This preparation is filled in a multi-dose eye drop container made of low density polyethylene (LDPE).

(Preparation Example 11) in 100 mL

| | |
|---|---|
| diquafosol sodium | 3 g |
| sodium hydrogen phosphate hydrate | 0.01 to 0.5 g |
| sodium chloride | 0.01 to 0.9 g |
| disodium edetate hydrate | 0.0001 to 0.1 g |
| polyvinylpyrrolidone K60 | 0.0001 to 10 g |
| silver nitrate | 0.0000001 to 0.01 g |
| hydroxyethylcellulose | 0.0001 to 5 g |
| pH adjustor | q.s. |
| purified water | q.s. |
| pH | 7.0 to 8.0 |

This preparation is filled in a multi-dose eye drop container made of low density polyethylene (LDPE).

(Preparation Example 12) in 100 mL

| | |
|---|---|
| diquafosol sodium | 3 g |
| sodium hydrogen phosphate hydrate | 0.01 to 0.5 g |
| sodium chloride | 0.01 to 0.9 g |
| disodium edetate hydrate | 0.0001 to 0.1 g |
| polyvinylpyrrolidone K30 | 0.0001 to 10 g |
| silver nitrate | 0.0000001 to 0.01 g |
| hydroxyethylcellulose | 0.0001 to 5 g |
| pH adjustor | q.s. |
| purified water | q.s. |
| pH | 7.0 to 8.0 |

This preparation is filled in a multi-dose eye drop container made of low density polyethylene (LDPE).

INDUSTRIAL APPLICABILITY

The present invention relates to an ophthalmic aqueous composition containing a silver salt, and filled in a container made of a polyester-based resin, or a container made of a polyolefin-based resin excluding polypropylene. The present ophthalmic aqueous composition has sufficient preservative efficacy over a long period of time, and hence can be formed into a multi-dose eye drop, and can be administered by instillation to an SCL wearing eye.

## Claims

1. An ophthalmic aqueous composition comprising a silver salt, the ophthalmic aqueous composition being filled in a container made of a polyester-based resin, or a container made of a polyolefin-based resin excluding polypropylene.

2. The ophthalmic aqueous composition according to claim 1, wherein the polyester-based resin is polyethylene terephthalate.

3. The ophthalmic aqueous composition according to claim 1, wherein the polyolefin-based resin is polyethylene.

4. The ophthalmic aqueous composition according to any one of claims 1 to 3, wherein a concentration of the silver salt in the ophthalmic aqueous composition is 0.001% (w/v) or less.

5. The ophthalmic aqueous composition according to any one of claims 1 to 4, wherein a concentration of the silver salt in the ophthalmic aqueous composition is 0.000003 to 0.0003% (w/v).

6. The ophthalmic aqueous composition according to any one of claims 1 to 4, wherein a concentration of the silver salt in the ophthalmic aqueous composition is 0.00001 to 0.0001% (w/v).

7. The ophthalmic aqueous composition according to any one of claims 1 to 4, wherein a concentration of the silver salt in the ophthalmic aqueous composition is 0.00002 to 0.0001% (w/v).

8. The ophthalmic aqueous composition according to any one of claims 1 to 7, further comprising an ionic tonicity agent.

9. The ophthalmic aqueous composition according to any one of claims 1 to 8, wherein the container made of a polyester-based resin or the container made of a polyolefin-based resin is a multi-dose eye drop container.

10. The ophthalmic aqueous composition according to any one of claims 1 to 9, wherein the ophthalmic aqueous composition is administered by instillation.

11. The ophthalmic aqueous composition according to any one of claims 1 to 10, wherein the ophthalmic aqueous composition is administered by instillation to a soft contact lens wearing eye.

12. The ophthalmic aqueous composition according to any one of claims 1 to 11, wherein the silver salt is silver nitrate.

13. The ophthalmic aqueous composition according to any one of claims 1 to 12, comprising an active ingredient.

14. The ophthalmic aqueous composition according to claim 13, wherein the active ingredient is rebamipide, diquafosol, or a salt thereof.

15. The ophthalmic aqueous composition according to claim 13, wherein the active ingredient is sirolimus or a salt thereof.

16. The ophthalmic aqueous composition according to claim 13, comprising an active ingredient excluding sirolimus or a salt thereof.

17. An ophthalmic aqueous composition comprising rebamipide, polyvinylpyrrolidone, and silver nitrate, the ophthalmic aqueous composition being filled in a multi-dose polyethylene eye drop container.

18. An ophthalmic aqueous composition comprising diquafosol sodium, polyvinylpyrrolidone, and silver nitrate, the ophthalmic aqueous composition being filled in a multi-dose polyethylene eye drop container.

19. An ophthalmic aqueous composition comprising sirolimus, a surfactant, and silver nitrate, the ophthalmic aqueous composition being filled in a multi-dose polyethylene eye drop container.

20. An ophthalmic aqueous composition comprising 0.00001 to 0.0001% (w/v) of silver nitrate, the ophthalmic aqueous composition being filled in a multi-dose polyethylene terephthalate eye drop container.

21. An ophthalmic aqueous composition comprising 0.00002 to 0.0001% (w/v) of silver nitrate, the ophthalmic aqueous composition being filled in a multi-dose polyethylene terephthalate eye drop container.

22. An ophthalmic preservative comprising a silver salt, the ophthalmic preservative being filled in a container made of a polyester-based resin or a container made of a polyolefin-based resin excluding polypropylene.

23. A method for imparting, to an ophthalmic aqueous composition, preservative efficacy satisfying a criterion of Preservatives-Effectiveness Tests of The Japanese Pharmacopoeia, comprising adding a silver salt to the ophthalmic aqueous composition, and filling the ophthalmic aqueous composition in a container made of a polyester-based resin or a container made of a polyolefin-based resin excluding polypropylene.

**INTERNATIONAL SEARCH REPORT**

International application No. PCT/JP2021/007366

A. CLASSIFICATION OF SUBJECT MATTER
Int. Cl. A61K47/02(2006.01)i, A61J1/05(2006.01)i, A61K9/08(2006.01)i, A61K31/4704(2006.01)i, A61K31/7084(2006.01)i, A61K47/32(2006.01)i, A61P27/02(2006.01)i
FI: A61K47/02, A61P27/02, A61K9/08, A61K31/4704, A61K31/7084, A61K47/32, A61J1/05 313B

According to International Patent Classification (IPC) or to both national classification and IPC

B. FIELDS SEARCHED

Minimum documentation searched (classification system followed by classification symbols)
Int. Cl. A61K47/02, A61J1/05, A61K9/08, A61K31/4704, A61K31/7084, A61K47/32, A61P27/02

Documentation searched other than minimum documentation to the extent that such documents are included in the fields searched
Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)
JSTPlus/JMEDPlus/JST7580 (JDreamIII); CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| Y | JP 2005-162648 A (ROHTO PHARMACEUTICAL CO., LTD.) 23 June 2005, claims 1-5, paragraphs [0023], [0028], [0033], [0041], [0050], [0063], [0067] | 1-23 |
| Y | JP 2005-330276 A (ROHTO PHARMACEUTICAL CO., LTD.) 02 December 2005, claim 5, paragraphs [0021], [0034], examples | 1-23 |
| Y | JP 2016-507469 A (SENJU PHARMACEUTICAL CO., LTD.) 10 March 2016, claims, paragraphs [0034]-[0036], [0041] | 1-23 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19.04.2021 | 27.04.2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2021/007366

C (Continuation). DOCUMENTS CONSIDERED TO BE RELEVANT

| Category* | Citation of document, with indication, where appropriate, of the relevant passages | Relevant to claim No. |
|---|---|---|
| Y | 新生児点眼剤 日本薬局方 硝酸銀点眼液 Silver Nitrate Ophthalmic Solution ボンハッピー. 医薬品インタビューフォーム. 2006, p. 1, "2. Characteristics and Utility of Product", non-official translation (Eyedrops for Newborns. Japanese Pharmacopoeia. Silver Nitrate Ophthalmic Solution. Bon Happy. Pharmaceutical Interview Form.) | 1-23 |
| Y | JP 2017-2036 A (SANTEN PHARMACEUTICAL CO., LTD.) 05 January 2017, claims, examples | 1-23 |
| Y | JP 2016-179960 A (ROHTO PHARMACEUTICAL CO., LTD.) 13 October 2016, claims, paragraph [0091] | 1-23 |
| Y | JP 2014-198709 A (SANTEN PHARMACEUTICAL CO., LTD.) 23 October 2014, claims, examples | 1-23 |
| Y | WO 2013/129318 A1 (ROHTO PHARMACEUTICAL CO., LTD.) 06 September 2013, claims, examples | 1-23 |
| Y | JP 2017-36255 A (ROHTO PHARMACEUTICAL CO., LTD.) 16 February 2017, claims, examples | 1-23 |
| X | WO 2020/004247 A1 (CSP ADVANCED SOLUTIONS INC.) 02 January 2020, claims 1, 4, 11 | 1, 3-7, 10, 12 |

Form PCT/ISA/210 (continuation of second sheet) (January 2015)

**INTERNATIONAL SEARCH REPORT**
Information on patent family members

International application No.
PCT/JP2021/007366

| Patent Documents referred to in the Report | Publication Date | Patent Family | Publication Date |
|---|---|---|---|
| JP 2005-162648 A | 23.06.2005 | (Family: none) | |
| JP 2005-330276 A | 02.12.2005 | (Family: none) | |
| JP 2016-507469 A | 10.03.2016 | US 2015/0366879 A1<br>claims, paragraphs [0034]-[0037], [0042]<br>EP 2956143 A1<br>KR 10-2015-0119303 A<br>CN 105228630 A | |
| JP 2017-2036 A | 05.01.2017 | US 2017/0348344 A1<br>claims, examples<br>KR 10-2017-0118255 A | |
| JP 2016-179960 A | 13.10.2016 | (Family: none) | |
| JP 2014-198709 A | 23.10.2014 | US 2016/0022648 A1<br>claims, examples<br>EP 2974728 A1<br>CN 105073112 A<br>KR 10-2015-0126345 A | |
| WO 2013/129318 A1 | 06.09.2013 | US 2013/0331458 A1<br>claims, examples<br>CN 104136019 A | |
| JP 2017-36255 A | 16.02.2017 | (Family: none) | |
| WO 2020/004247 A1 | 02.01.2020 | US 2020/0000913 A1<br>claims 1, 4, 11 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017002036 A
- JP 2016507469 A